# EUROPÄISCHE PATENTSCHRIFT

(11) **EP 0 358 056 B1**
(45) Veröffentlichungstag und Bekanntmachung des Hinweises auf die Patenterteilung: **20.10.1993**
(21) Anmeldenummer: 89115623.4
(22) Anmeldetag: 24.08.1989
(51) Int. Cl.: A61F 2/64

(54) **Kniegelenk für eine Beinprothese**
Knee joint for a leg prosthesis
Articulation du genou pour une prothèse de jambe

(30) Priorität: 07.09.1988 DE 3830330
(43) Veröffentlichungstag der Anmeldung: 14.03.1990
(73) Patentinhaber: Schütt & Grundei Orthopädietechnik GmbH, D-23556 Lübeck (DE)
(72) Erfinder: Grundei, Hans, D-2400 Lübeck (DE)
(74) Vertreter: Fuchs Mehler Weiss

(56) Entgegenhaltungen:
- AT-B- 323 318
- BE-A- 564 296
- DE-C- 490 884
- DE-C- 828 292
- DE-C- 843 880
- GB-A- 157 260

## Beschreibung

Die Erfindung betrifft ein Kniegelenk für eine Beinprothese gemäß dem Oberbegriff des Anspruchs 1.

Bei bekannten Gelenken für Beinprothesen besteht die Gelenkverbindung zwischen Ober- und Unterteil aus einem Scharnier, durch welches der Unterteil und Oberteil relativ zueinander verschwenkbar sind. Diese Beinprothesen lassen also nur eine scharnierartige Schwenkbewegung zu. Solche einfachen Schwenkbewegungen stimmen verständlicherweise nicht mit den Bewegungen eines natürlichen Kniegelenks überein.

Aus der DE-C-828292 ist ein gattungsgemäßes Kniegelenk bekannt. Bei dem darin beschriebenen Kunstbein handelt es sich um ein übliches Holzbein, bei dem Ober- und Unterteil scharnierartig zueinander verschwenkbar sind. Hierzu sind die Köpfe des Oberteils entsprechend den Pfannenflächen des Unterteils ausgebildet, in denen sie bei Ausführung der Beugebewegung zwangsgeführt sind. Hieraus ergibt sich, daß bei diesem Gelenk keine Rotationsmöglichkeit zwischen dem Ober- und Unterteil besteht. Ein weiterer Nachteil ist bei dieser Konstruktion darin zu sehen, daß die Gelenkteile von Gelenkachsen durchsetzt sind, welche die gesamten Kräfte eines Lastwechsels aufzunehmen haben mit der mutmaßlichen Folge, daß die Achsen rasch ausschlagen. Es ist aus dieser Druckschrift auch nicht ersichtlich, daß bei diesem Kniegelenk eine kombinierte Wälz- und Gleitbewegung zwischen den Gelenkteilen stattfindet, wie dies bei einem natürlichen Kniegelenk der Fall ist. Entgegen dem physiologischen Beuregungsablauf eines natürlichen Unies bewegt sich bei dem darin beschriebenen Gelenk mit zunehmender Beugung das Unterteil gegenüber dem Oberteil nach hinten.

In etwa vergleichbare Konstruktionen zeigen die DE-C-843880 und DE-C-490884. Die darin beschriebenen Gelenkteile weisen dieselben Nachteile auf wie jenes gemäß der DE-C-828292.

Darüber hinaus ist aus der AT-A-323 318 ein Kniegelenk bekanntgeworden, das als Doppelachsgelenk ausgeführt ist, bei dem das Oberteil auf einer vorgegebenen Zwangskurve gegenüber dem Unterteil geführt wird. Durch die Festlegung der Achsen ist eine erwähnte Rückschubbewegung des Oberteils gegenüber dem Unterteil sowie eine Rotationsbewegung zwischen beiden Teilen nicht möglich, so daß auch mit diesem Kniegelenk ein möglichst physiologischer Bewegungsablauf nicht erzielbar ist.

Vor diesem Hintergrund ist es die Aufgabe der vorliegenden Erfindung, ein Kniegelenk für eine Beinprothese der eingangs genannten Art zu schaffen, bei dem die Beugebewegung des Unterteils zum Oberteil weitgehend den Beugebewegungen eines natürlichen Kniegelenks entsprechen.

Diese Aufgabe wird erfindungsgemäß durch die im Anspruch 1 angegebenen kennzeichnenden Merkmale gelöst. Besondere Ausführungsarten der Erfindung sind in den Unteransprüchen angegeben. Durch diese Ausbildung wird praktisch eine einem natürlichen Kniegelenk entsprechende Beugebewegung des Unterteils zum Oberteil erreicht. Dabei werden die Kufen durch die Federwirkung in jeder Beugelage gegen die Gleitflächen gehalten, so daß beim Gehen mit der Beinprothese die Kufen stets auf den Gleitflächen verbleiben und beim Gehen keine Stöße auftreten.

Ein Abgleiten der Kufen durch seitliches Abknicken des Unterteils gegenüber dem Oberteil wird dadurch verhindert, daß seitlich am Rahmen geführte Laschen vorgesehen sind, die mit dem Oberteil gelenkig verbunden sind. Weitere vorteilhafte Merkmale des Gelenks sind in den Unteransprüchen angegeben.

Die Erfindung wird nachstehend anhand eines in der anliegenden Zeichnung dargestellten Ausführungsbeispieles naher erläutert.

Es zeigt:

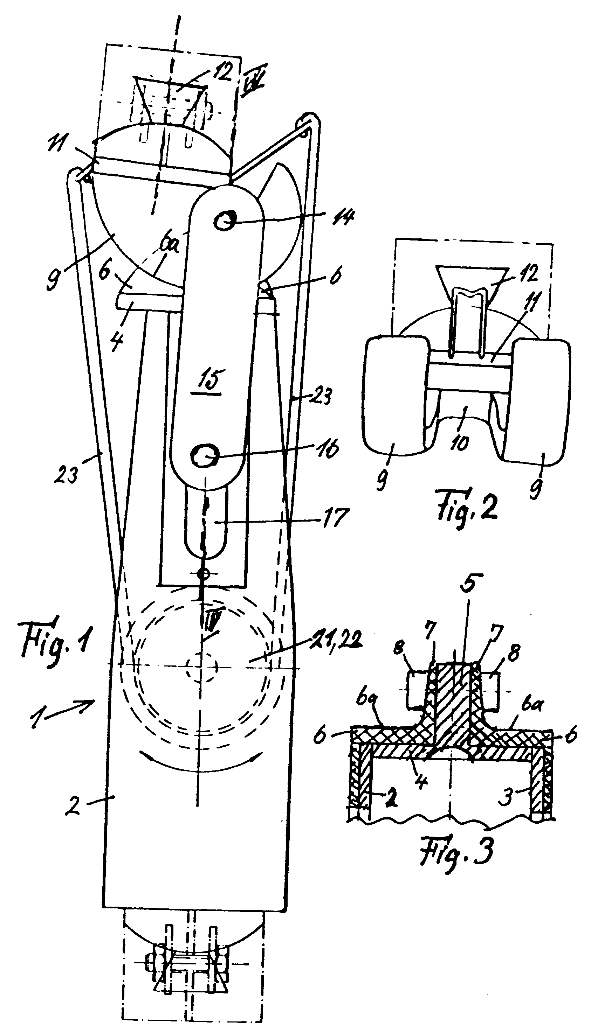

- Figur 1: eine Seitenansicht des Gelenks,
- Figur 2: die Rückansicht des Oberteils im Bereich der Kufen,
- Figur 3: einen Querschnitt des Unterteils im Bereich der Gleitflächen und

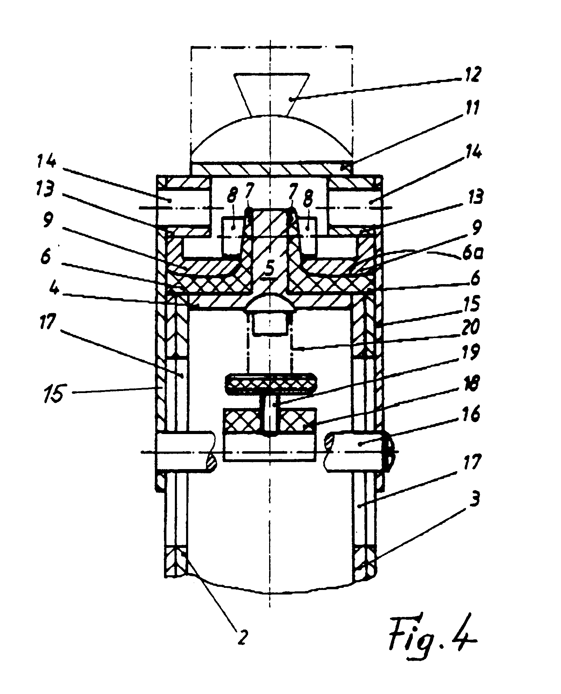

- Figur 4: einen Schnitt nach der Schnittlinie IV-IV der Figur 1.

Das Gelenk ist mit einem Rahmen 1 ausgerüstet, mit dessen Unterende ein künstlicher Unterschenkel mit Fuß verbindbar ist. Dieser Rahmen 1 ist rechteckig ausgebildet und hat zwei parallele aufrechte Schenkel 2 und 3, die oben durch eine Platte 4 verbunden sind. Die Schenkel 2, 3 sind auch unten verbunden und mit üblichen Teilen zum Anschluß des künstlichen Unterschenkels versehen.

Die obere Platte 4 des Rahmens ist mit einer nach oben verlaufenden Rippe 5 versehen und dient zur festen Unterstützung eines Trägers 6 mit den beiden Gleitflächen 6a aus abriebfestem Kunststoff.Die Abwinkelungen 7 am Träger 6 fassen die Rippe 5 beidseitig ein und tragen waagerechte seitwärts gerichtete Zapfen 8, deren Funktion noch erläutert wird.

Auf den Gleitflächen 6a stützen sich zwei Kufen 9 mit von vorn nach hinten verlaufenden,in Seitenansicht im wesentlichen elliptischen Anlageflächen ab. Die Seitenwände der Kufen 9 sind vorn bei 10 überbrückt und mit einer oberen Platte 11 versehen, die exzentrisch mit einem Kupplungszapfen 12 versehen ist, mit dem eine Verbindung zu einer nicht gezeigten Aufnahme für den Oberschenkelstumpf kuppelbar ist und dessen Achse nach vorn exzentrisch zur Achse des Unterteiles versetzt ist.

Die Platte 11 ist auf der hinteren Unterseite mit seitlichen Lagern 13 für den Eingriff zweier Zapfen 14 versehen, die in seitlichen Laschen 15 befestigt sind, welche außen an den Schenkeln 2 und 3 des Rahmens 1 anliegend geführt sind.

Die Unterenden der Laschen 15 sind durch eine Strebe 16 miteinander verbunden, die Langlöcher 17 der Rahmenschenkel 2,3 durchgreift. Die Strebe 16 trägt einen Sockel 18, in den eine Schraube 19 gedreht ist, gegen deren Kopf sich das Unterende einer Feder 20 abstützt. Durch Verdrehung der Schraube 19 ist die Spannung der Feder 20 einstellbar.

Durch die Laschenverbindung zwischen dem Oberteil und dem Unterteil und durch die Feder 20 werden die Kufen 9 bei jeder Beugebewegung des Unterteiles zum Oberteil mit ihren Anlageflächen stets gegen die Gleitflächen 6a gehalten, so daß beim Gehen des Patienten die Kufen auf den Gleitflächen bleiben. Die Kufen 9 werden zusätzlich durch die Zapfen 8 des Unterteiles geführt, die auf den Kufen liegen, wobei die oberen Anlageflächen an den Kufen 9 für die Zapfen 8 parallel zu den unteren Auflageflächen der Kufen 9 verlaufen, mit denen sich die Kufen auf den Gleitflächen 6a abstützen.

Zur Einleitung und Unterstützung der Beugebewegung ist im Rahmen 1 ein elektrischer Umkehrmotor 21 mit einer Zahnscheibe 22 vorgesehen, über die ein gezahnter Riemen 23 verläuft, dessen Enden vorn und hinten mit dem Oberteil verbunden sind. Über Sensoren bzw. Elektroden ist der Motor mit Restmuskeln (Beuge- und Streckmuskel) des Oberschenkels verbunden, durch die in bekannter Weise Kontakte für den Vorwärts- und Rückwärtsantrieb des Motors betätigt werden und die Beugung des Unterteiles zum Oberteil nach hinten und nach vorn ausgelöst wird.

## Patentansprüche

1. Kniegelenk für eine Beinprothese mit einem Oberteil, der mit der Aufnahme für den Oberschenkelstumpf des Patienten verbindbar ist, und mit einem gelenkig mit dem Oberteil verbundenen Unterteil, der mit der Nachbildung eines Teiles des Unterschenkels und des Fußes verbindbar ist, wobei sich der Oberteil mit Kufen auf Gleitflächen des Unterteils abstützt und die Kufen durch Federwirkung gegen die Gleitflächen gehalten werden und wobei der Oberteil mit den Kufen auf den Gleitflächen bis zum Erreichen einer Strecklage um einen Schwenkpunkt schwenkbar ist, wobei die vertikale Hauptachse des Oberteils (9-12) gegenüber der vertikalen Hauptachse des Unterteils nach vorne versetzt ist, dadurch gekennzeichnet, daß die Kufen (9)in Seitenansicht im wesentlichen Anlageflächen aufweisen, die von vorm nach hinten gesehen um den Schwenkpunkt (14) stetig kleinet werdende Krümmungsradien einnehmen und die auf den im wersentlichen flach ausgebildeten Gleitflächen (6a) des Unterteils aufliegen, wobei sich das Oberteil gegenüber dem Unterteil bei zunehmen der Beugung des Gelenks nach hinten verlagert.

2. Kniegelenk nach Anspruch 1, dadurch gekennzeichnet, daß der Unterteil einen Rahmen (1) aufweist, der einen die Gleitflächen (6a) aufweisenden Träger (6) abstützt, daß der Oberteil (9-12) durch Achszapfen (14) mit seitlich am Rahmen (1) geführten Laschen (15) verbunden ist, die durch eine Langlöcher (17) durchgreifende Strebe (16) starr verbunden sind und daß die Strebe (16) durch eine Feder (20) gegenüber dem Rahmen (1) abgefedert ist und über die Laschen (15) die Kufen (9) gegen die Gleitflächen (6a) des Unterteils hält.

3. Gelenk nach Anspruch 1 oder 2, dadurch gekennzeichnet, daß die Spannung der Feder (20) einstellbar ist.

4. Gelenk nach Anspruch 3, dadurch gekennzeichnet, daß die Feder (20) eine Druckfeder ist, deren eines Ende sich gegen die Rahmenstütze (4) für den Träger (6) abstützt und deren anderes Ende sich gegen eine Schraube (19) abstützt, die in einem mit der Strebe (16) verbundenen Sockel (18) verschraubt ist.

5. Gelenk nach einem der Ansprüche 1 bis 4, dadurch gekennzeichnet, daß feststehende Zapfen (8) des Unterteils bei Beugebewegungen an parallel zu den Kufenflächen verlaufenden Innenflächen der Kufen (9) als Führung für die Kufen gleiten.

6. Gelenk nach einem der Ansprüche 1 bis 5, dadurch gekennzeichnet, daß im Rahmen (1) ein Elektromotor (21) mit Zahnscheibe (22) auf seiner Achse montiert ist, daß über die Zahnscheibe ein gezahnter Treibriemen (23) verläuft, dessen Enden vorn und hinten am Oberteil angreifen, und daß über Sensoren (Impulselektroden), welche mit Restmuskeln (Beuge- und Streckmuskeln) des Oberschenkels Verbindung haben, Kontakte für den Vorwärts- oder Rückwärtslauf des Motors augelöst werden.

## Claims

1. Knee joint for a leg prostheses comprising an upper part which can be connected with the receptacle for the above-knee stump of the patient, and a lower part which is connected with the upper part in articulated relationship and which can be connected with the imitation of a part of the lower leg and with the foot, the upper part being supported on slide faces of the lower part by means of rockers and said rockers being held against said slide faces by means of springiness and wherein the upper part can be swiveled with the rockers and the slide faces round a swivel center until a stretch position has been reached, the vertical principal axis of the upper part (9-12) being staggered forward with respect to the vertical principal axis of the lower part, characterized in that, in lateral view, the rockers (9) essentially comprise contact surfaces which, in view from front to back, show continually decreasing radii of curvature around the swivel center (14) and which are supported by said essentially flat developed slide faces (6a) of the lower part, whereas the upper part shifts backward with respect to the lower part when the joint continues to be bent.

2. Knee joint according to claim 1, characterized in that the lower part comprises a frame (1) strutting a support which comprises the slide faces (6a), that the upper part (9-12) is connected with shackles (15) being guided at the side of said frame (1), said shackles being rigidly connected by means of a prop (16) passing through slotted holes (17), and in that said prop is spring-loaded against the frame (1) by means of a spring (20) and holds up the rockers (9) against the slide faces (6a) of the lower part by means of said shackles (15).

3. Knee joint according to claims 1 or 2, characterized in that the tension of the spring (20) is adjustable.

4. Knee joint according to claim 3, characterized in that the spring (20) constitutes a compression spring, one end of which is braced with the frame base (4) for the support (6) and the other end of which is braced with a screw (19) being screwed on a base (18) connected with said prop (16).

5. Knee joint according to one of claims 1 to 4, characterized in that, in performing bending motions, as guideways for the rockers, stationary journals (8) of the lower part slide along inner surfaces of the rockers (9) running in parallel to the rocker sides.

6. Knee joint according to one of claims 1 to 5, characterized in that an electric motor (21) having a toothed disk (22) on its wheel shaft is mounted in the frame (1), that a toothed driving belt (23) runs over said toothed disk, the ends of said driving belt engaging in front and at the back of the upper part and in that contacts for forward and reverse motion of the motor are made via sensors (impulse electrodes) being connected with residual muscles (flexors and extensors) of the above-knee.

## Revendications

1. Articulation du genou pour une prothèse de jambe, avec une partie supérieure, susceptible d'être reliée au logement destiné au moignon de fémur supérieur du patient et avec une partie inférieure reliée de façon articulée à la partie supérieure et susceptible d'être reliée à une partie simulant la jambe et le pied, la partie supérieure prenant appui, par des patins, sur des surfaces de glissement de la partie inférieure et les patins étant maintenus, par un effet élastique, contre les surfaces de glissement, et la partie supérieure dotée des patins étant susceptible de pivoter, autour d'un centre de pivotement, sur les surfaces de glissement jusqu'à atteinte d'une position étendue, l'axe principal vertical de la partie supérieure (9 à 12) étant décalé vers l'avant par rapport à l'axe principal vertical de la partie inférieure, caractérisée en ce que les patins (9) présentent en vue de côté essentiellement des surfaces d'appui qui, en observant de l'avant vers l'arrière, ont des rayons de courbure devenant toujours de plus en plus petits, autour du point de pivotement (14) et appuient sur les surfaces de glissement (6a) pratiquement planes de la partie inférieure, la partie supérieure se déplaçant vers l'arrière par rapport à la partie inférieure lorsque la flexion de l'articulation augmente.

2. Articulation du genou selon la revendication 1, caractérisée en ce que la partie inférieure présente un cadre (1) servant d'appui à un support (6) présentant les surfaces de glissement (6a), en ce que la partie supérieure (9 à 12) est reliée, au moyen de tourillons en forme d'axe (14), à des pattes (15) guidées latéralement sur le cadre (1) et reliées rigidement, au moyen d'une fiche (16) traversant des trous allongés (17), et en ce que la fiche (16) est poussée élastiquement par rapport au cadre (1), au moyen d'un ressort (20) et maintient les patins (9) contre les surfaces de glissement (6a) de la partie inférieure par l'intermédiaire des pattes (15).

3. Articulation selon la revendication 1 ou 2, caractérisée en ce que la tension du ressort (20) est réglable.

4. Articulation selon la revendication 3, caractérisée en ce que le ressort (20) est un ressort de pression, dont une extrémité prend appui contre la traverse de cadre (4) destiné au support (6) et dont l'autre extrémité prend appui contre une vis (19) vissée dans une embase (18) reliée à la fiche (16).

5. Articulation selon l'une des revendications 1 à 4, caractérisée en ce que les tourillons fixes (8) de la partie inférieure glissent, à titre de guidage des patins, lors des mouvements de flexion, sur les surfaces intérieures, s'étendant parallèlement aux surfaces des patins (9).

6. Articulation selon l'une des revendications 1 à 5, caractérisée en ce que dans le cadre (1) est monté un moteur électrique (21) présentant sur son axe un disque denté (22), en ce que sur le disque denté passe une courroie de transmission crantée (23), dont les extrémités agissent à l'avant et à l'arrière de la partie supérieure, et en ce que par l'intermédiaire de capteurs (électrodes à impulsion) reliés aux muscles restants (muscles de flexion et d'extension) de la cuisse, sont déclenchés des contacts destinés au fonctionnement en marche avant ou en marche arrière du moteur.
